# EUROPÄISCHE PATENTSCHRIFT

(11) **EP 0 749 735 B1**
(45) Veröffentlichungstag und Bekanntmachung des Hinweises auf die Patenterteilung: **22.08.2001**
(21) Anmeldenummer: 95810419.2
(22) Anmeldetag: 21.06.1995
(51) Int. Cl.: A61F 2/42

(54) **Künstliches Handgelenk**
Artificial hand joint
Articulation artificielle de la main

(43) Veröffentlichungstag der Anmeldung: 27.12.1996
(73) Patentinhaber: Sulzer Orthopädie AG, 6340 Baar (CH)
(72) Erfinder: Lamprecht, Stefan, CH-8309 Birchwil (CH)
(74) Vertreter: Sulzer Management AG

(56) Entgegenhaltungen:
- EP-A- 0 607 749
- GB-A- 2 269 752

## Beschreibung

Die Erfindung betrifft ein künstliches Handgelenk entsprechend dem Oberbegriff des Patentanspruchs 1.

Ein aus der EP-A-0 607 749 bekanntes künstliches Handgelenk der genannten Art enthält einen distalen sphärischen Tragkopf und ein proximales Gegenstück mit einer rinnenförmigen Führungsbahn für den Tragkopf. Die bekannte Ausführung lässt Flexions- und Extensionsbewegungen zu, wobei der Bereich der Extensionsbewegungen jeweils durch zwei die Längsverschiebungen des Tragkopfs begrenzende, hochgezogene Endpartien der Führungsbahn bestimmt ist. Innerhalb des so begrenzten Bewegungsbereichs ist der Tragkopf jedoch in Richtung der Extensionsbewegungen ungeführt, so dass er in Längsrichtung der Führungsbahn seitliche Kippbewegungen ausführen und damit die mit dem Tragkopf verbundene Handpartie seitlich ungeführte, lediglich durch die Bänder und Sehnen der Handpartie begrenzte, unbestimmte Winkelstellungen bezüglich der Längsachse des Radius einnehmen kann. Der Tragkopf und das Gegenstück der bekannten Gelenkanordnung sind ferner je mit einem im entsprechenden Knochen zu verankernden zentralen Zapfen ausgeführt, der mit Längsrippen versehen ist. Diese Zapfen erfordern je eine relativ voluminöse Ausführung und damit entsprechend grosse, in die Knochen einzuarbeitende und deren Querschnitt schwächende Ausnehmungen zur Aufnahme dieser Verankerungsteile. Um bei der bekannten Gelenkanordnung ungehinderte Flexions- und Extensionsbewegungen der zusammenwirkenden Teile des Handgelenks zu gewährleisten, müssen Tragkopf und Führungsbahn je mit einer relativ grossen Auflagefläche ausgeführt sein, welche eine in Richtung der Flexionsbewegung entsprechend breite Ausführung des Gegenstücks und damit einen entsprechend grossen, am Radius zu schaffenden Implantationsbereich erfordert.

Der Erfindung liegt die Aufgabe zugrunde, ein insbesondere in dieser Hinsicht verbessertes, weiter entwickeltes Implantat der eingangs genannten Art in einer kompakten Bauweise zu schaffen, welche eine sichere Anbringung der zusammenwirkenden Teile im Implantationsbereich mit möglichst geringem Verlust an Knochenmaterial ermöglicht und welche ungehinderte Flexions- und Extensionsbewegungen der zusammenwirkenden Teile sowie eine verbesserte Führung des distalen Gelenkteils innerhalb seines in Richtung der Extensionsbewegung verlaufenden Bewegungsbereichs ermöglicht.

Diese Aufgabe wird erfindungsgemäss durch die im kennzeichnenden Teil des Patentanspruchs 1 angegebenen Merkmale gelöst.

Das erfindungsgemäss ausgeführte Handgelenk ermöglicht unbehinderte Flexions- und Extensionsbewegungen, wobei die beiden Tragköpfe eine Seitenstabilität des distalen Gelenkteils bei den Extensionsbewegungen, und damit jeweils eine definierte Winkelposition des distalen Handbereichs bezüglich der Längsachse des Radius gewährleisten. Die Stützflächen der beiden Tragköpfe können je mit einem relativ kleinen Krümmungsradius ausgeführt werden, so dass die Führungsbahn sowie das die Tragköpfe aufnehmende Führungsstück mit entsprechend geringer Breite ausgeführt werden können und damit der durch Entfernen von Knochenmaterial zu bildende Implantationsbereich klein gehalten und der Eingriff in den Knochenbereich des Handgelenks minimiert werden kann. Ein weiterer Vorteil der erfindungsgemässen Ausführung besteht darin, dass die Implantate je in einer leichten Bauweise, mit geringen Abmessungen hergestellt werden können, welche trotzdem eine sichere Verankerung der Gelenkteile in den Knochen des Handbereichs gewährleisten.

Ausgestaltungen der Erfindung sind in den abhängigen Patentansprüchen angegeben.

Die Erfindung wird anhand von in der Zeichnung schematisch dargestellten Ausführungsbeispielen erläutert. Es zeigen:
- Fig.1: einen Ausschnitt eines Handgelenks in einer schematischen Draufsicht in einer von Radius und Ulna bestimmten Ebene, mit Gelenkteilen in einem Teilschnitt,
- Fig.2 und 3: die Gelenkteile in einer grösseren Darstellung und je in einer Ansicht gemäss Pfeil II - II bzw. III - III in Fig.1,
- Fig.4: entsprechende Gelenkteile eines Handgelenks nach einer abgewandelten Ausführungsform, in einer der Fig.2 entsprechenden Darstellung,
- Fig.5: einen Ausschnitt eines Handgelenks in einer Teildraufsicht und in einen Teilschnitt, mit Gelenkteilen nach einer weiteren abgewandelten Ausführungsform, und
- Fig.6: die Gelenkteile in einer Seitenansicht gemäss Pfeil VI in Fig.5.

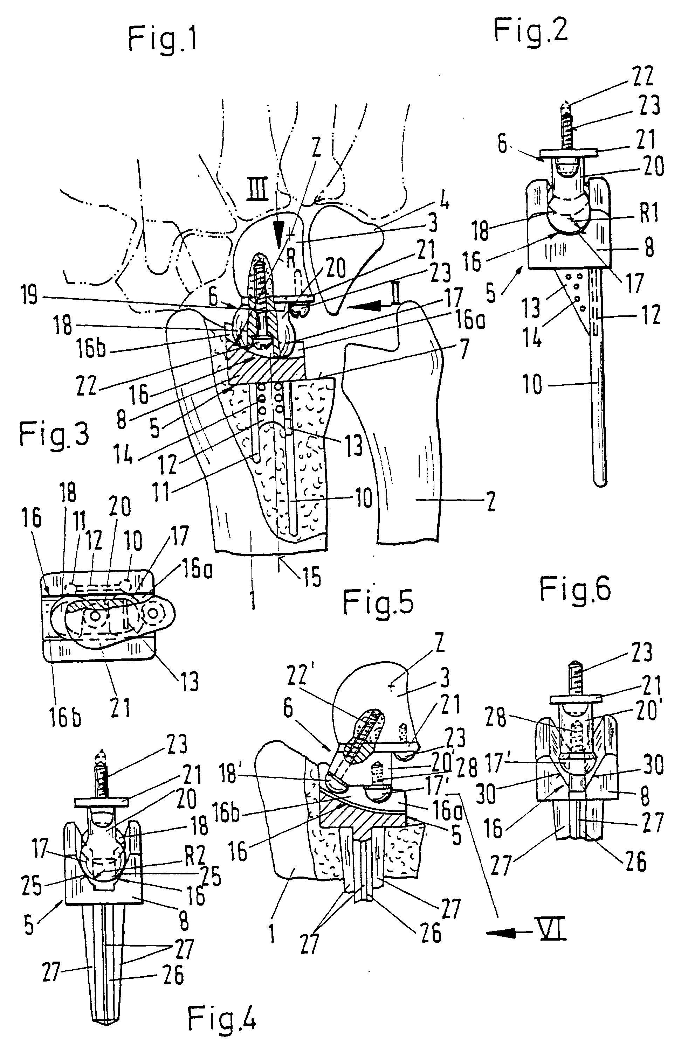

Die Fig.1 zeigt einen an zwei Unterarmknochen - Radius 1 und Ulna 2 - anschliessenden Handbereich, in welchem zwei distale Karpalknochen - Capitate 3 und Hamate 4 - mit vollen Linien und weitere, unveränderte Handknochen mit strichpunktierten Linien dargestellt sind und in welchem die proximalen Karpalknochen entfernt worden sind. Das künstliche Handgelenk umfasst einen am Radius 1 befestigbaren proximalen Gelenkteil 5 und einen am Capitate 3 befestigbaren distalen Gelenkteil 6. Der proximale Gelenkteil 5 enthält ein auf eine Resektionsfläche 7 des Radius 1 aufsetzbares Führungsstück 8 mit zwei davon abstehenden Befestigungselementen in Form von im Radius 1 je in einer entsprechenden Bohrung verankerbaren Stiften 10 und 11, welche über in den Knochen einführbare Rippen 12 und 13 miteinander und mit dem Führungsstück 8 biegesteif verbunden sind. Die Rippen 12 und 13 können darstellungsgemäss mit Durchtrittsöffnungen 14 versehen sein, welche ein Einwachsen von Knochenmaterial ermöglichen und damit, bei geringstmöglichem Eingriff in den Knochenbereich, eine sichere Verankerung der Befestigungselemente im Radius 1 gewährleisten. Das Führungsstück 8 ist mit einer quer zu einer Längsachse 15 des Radius 1 verlaufenden, in Richtung einer Extensionsbewegung des Handgelenks orientierbaren rinnenförmigen Führungsbahn 16 ausgeführt, in welcher der distale Gelenkteil 6 entsprechend einer durch Radius 1 und Ulna 2 bestimmten Ebene verschiebbar geführt ist. Die Führungsbahn 16 ist in Form einer Nut mit einem halbkreisförmigen Querschnitt und einer über ihre Längserstreckung bogenförmig verlaufenden Grundfläche ausgeführt, die darstellungsgemäss durch einen Krümmungsradius R bestimmt ist, dessen ideeller Drehpunkt Z nach der Implantation der Gelenkteile 5 und 6 im Bereich des Capitate 3 liegen kann.

Der distale Gelenkteil 6 ist mit zwei in Richtung der Extensionsbewegung im Abstand voneinander angeordneten, in die Führungsbahn 16 einführbaren Tragköpfen 17 und 18 ausgeführt, welche über eine gemeinsame Halspartie 20 mit einer auf eine Resektionsfläche 19 des Capitate 3 aufsetzbaren Tragplatte 21 verbunden und mittels Schrauben 22, 23 am Capitate 3 befestigbar sind. Die Tragköpfe 17 und 18 sind darstellungsgemäss in Form von gegenüber der Halspartie 20 verdickten, an dieser angeformten Fortsätzen mit sphärischen Stützflächen ausgeführt, welche je unter Linienberührung mit einem entsprechenden Abschnitt 16a bzw. 16b der Führungsbahn 16 zusammenwirken. Der Querschnitt der Führungsbahn 16 ist mit einem Radius R1 ausgeführt, der um ein eine Gleitführung zulassendes Spiel grösser ist als die Radien der Stützflächen der Tragköpfe 17 und 18.

Die Gelenkteile 5 und 6 können je mehrteilig oder, wie dargestellt, als einstückige Bauteile ausgebildet sein, von denen z.B. ein Vorrat von einigen aufeinander abgestimmten Ausführungen mit unterschiedlichen, abgestuften Abmessungen bereitgestellt werden kann. Nach der Implantation der Gelenkteile 5 und 6, wenn diese durch nicht dargestellte Bänder und Sehnen des Bandapparats in der anatomisch richtigen Position gehalten werden, wird durch die beiden Tragköpfe 17 und 18 eine in Richtung der Extensionsbewegung stabile Führung der zusammenwirkenden Gelenkteile 5 und 6 erzielt, indem die beiden Tragköpfe 17 und 18 je auf dem zugehörigen Abschnitt 16a bzw. 16b der Führungsbahn 16 verschiebbar abgestützt sind. Zugleich wird die Beweglichkeit der beiden Tragköpfe 17 und 18 in Richtung der Flexionsbewegungen gewährleistet, da der halbkreisförmige Querschnitt der Führungsbahn 16, in Verbindung mit der gegenüber den Tragköpfen 17, 18 verjüngten Halspartie 20, relativ grosse Schwenkbewegungen des distalen Gelenkteils 6 quer zur Längserstreckung der Führungsbahn 16 zulässt.

Entsprechend der Darstellung nach Fig.4 kann die Führungsbahn 16 durch V-förmig angeordnete seitliche Flanken 25 begrenzt sein, welche, im Querschnitt gesehen, je mit einem Krümmungsradius R2 ausgeführt sind, der grösser ist als die Krümmungsradien der an den Tragköpfen 17 und 18 ausgebildeten sphärischen Stützflächen. Entsprechend sind die beiden Tragköpfe 17 und 18 je über zwei im wesentlichen punktförmige Aufsetzpartien auf den Flanken 25 der Führungsbahn 16 beweglich abgestützt, wodurch ebenfalls eine stabile Führung der zusammenwirkenden Gelenkteile 5 und 6 in Richtung der Extensionsbewegungen erzielt werden kann. Zugleich wird die Beweglichkeit der Tragköpfe 17 und 18 quer zur Längserstreckung der Führungsbahn 16 gewährleistet, da diese mit einer relativ grossen Breite und Tiefe ausgeführt werden kann, wodurch ein entsprechend grosser Schwenkbereich der zusammenwirkenden Gelenkteile 5 und 6 in Richtung der Flexionsbewegungen erzielbar ist. Wie aus der Fig.4 weiter hervorgeht, kann das Führungsstück 8 auch mit einem im Radius 1 verankerbaren zentralen Zapfen 26 ausgeführt sein, der mit mehreren, darstellungsgemäss vier, von ihm radial abstehenden, in entsprechende Schlitze der Knochenpartie einführbaren Führungsrippen 27 versehen sein kann.

Gemäss Fig.5 kann der distale Gelenkteil 6 mit einer am Capitate 3 befestigbaren Halterung 20' und zwei je für sich an dieser anbringbaren Tragköpfen 17' und 18' ausgeführt sein, die darstellungsgemäss je durch einen halbkugelförmigen Schraubenkopf einer in der Halterung 20' verankerbaren Schraube 28 bzw. einer die Halterung 20' durchsetzenden, im Capitate 3 verankerbaren Schraube 22' gebildet sind. Wie aus der Fig.5 weiter hervorgeht, können die Tragköpfe 17' und 18' mit unterschiedlichen Abmessungen ausgeführt und mit entsprechend unterschiedlichen Eindringtiefen in der Führungsbahn 16 angeordnet sein. Durch geeignete Wahl der Tragköpfe 17' und 18' kann die Winkelstellung der Halterung 20' bezüglich der Führungsbahn 16 beeinflusst und damit an die jeweils gegebenen Verhältnisse im Anschlussbereich des Capitate 3 angepasst werden.

Gemäss Fig.6 kann die Führungsbahn 16 des Führungsstücks 8 auch durch zwei gegen ihre bogenförmig verlaufende Grundfläche konvergierende seitliche Flanken 30 begrenzt sein, die je in Form einer Mantelfläche eines Kegelstumpfs ausgeführt sind. Nach einer abgewandelten, nicht dargestellten Ausführungsform des distalen Gelenkteils 6 kann auch nur einer der Tragköpfe 17' oder 18' als an der Halterung 20' befestigbarer Stützteil, und der andere Tragkopf 18' bzw. 17' als an der Halterung 20' angeformter Fortsatz ausgebildet sein.

Zusammenfassend lässt sich die Erfindung wie folgt beschreiben:

Das künstliche Handgelenk umfasst einen am Radius befestigbaren proximalen Gelenkteil mit einer rinnenförmigen Führungsbahn und einen am Capitate befestigbarem distalen Gelenkteil mit zwei nebeneinander angeordneten, in der Führungsbahn in Richtung ihrer Längserstreckung verschiebbar und in Querrichtung neigbar gelagerten Tragköpfen. Diese sind mit sphärischen Stützpartien ausgeführt, welche je mit einem Führungsabschnitt der Führungsbahn zusammenwirken. Durch die beiden Tragköpfe wird eine in Richtung von Extensionsbewegungen stabile Führung des distalen Gelenkteils erzielt. Die Führungsbahn und der proximale Gelenkteil können mit vorteilhaft geringen Querschnittsabmessungen ausgeführt werden, so dass die Eingriffe in den Implantationsbereich des Handgelenks minimiert werden können.

## Patentansprüche

1. Künstliches Handgelenk zum Ausführen einer Flexions- und einer Extensionsbewegung, mit einem am Capitate (3) befestigbaren distalen Gelenkteil (6), der einen Tragkopf (17, 17') mit einer balligen Stützfläche enthält, und einem am Radius (1) befestigbaren proximalen Gelenkteil (5), welcher eine zur Aufnahme des Tragkopfs (17, 17') bestimmte Führungsbahn (16) mit einem quer zur Längsachse (15) des Radius (1), in Richtung der Extensionsbewegung verlaufenden rinnenförmigen Führungsabschnitt (16a) enthält, in welchem der Tragkopf (17, 17') in einer durch Radius (1) und Ulna (2) bestimmten Ebene verschiebbar geführt ist,
dadurch gekennzeichnet, dass der distale Gelenkteil (6) einen in Richtung der Extensionsbewegung neben dem einen, ersten Tragkopf (17, 17') angeordneten zweiten Tragkopf (18, 18') mit einer zweiten balligen Stützfläche enthält, und dass die Führungsbahn (16) des proximalen Gelenkteils (5) mit einem zur Aufnahme des zweiten Tragkopfs (18, 18') bestimmten zweiten Führungsabschnitt (16b) ausgeführt ist.

2. Handgelenk nach Anspruch 1, dadurch gekennzeichnet, dass der distale Gelenkteil (6) eine am Capitate (3) mit einem Befestigungsmittel (22, 22', 23) verankerbare Halspartie (20, 20', 21) aufweist und dass die beiden Tragköpfe (17, 18; 17', 18') je durch einen von der Halspartie (20, 20', 21) proximal abstehenden, zumindest teilweise kugelförmigen Vorsprung gebildet sind.

3. Handgelenk nach Anspruch 2, dadurch gekennzeichnet, dass die beiden Tragköpfe (17, 18) je an einem an der Halspartie (20, 21) angeformten Fortsatz ausgebildet sind.

4. Handgelenk nach Anspruch 1 oder 2, dadurch gekennzeichnet, dass mindestens einer der beiden Tragköpfe (17', 18') durch ein für sich am Capitate (3) - und/oder an der Halspartie (20') - befestigbares Stützelement gebildet ist.

5. Handgelenk nach einem der Ansprüche 1 bis 4, dadurch gekennzeichnet, dass die Führungsbahn (16) des proximalen Gelenkteils (5), im Querschnitt gesehen, mit entsprechend den Stützflächen der Tragköpfe (17, 18) gekrümmten Führungsflächen ausgeführt ist.

6. Handgelenk nach einem der Ansprüche 1 bis 4, dadurch gekennzeichnet, dass die Führungsbahn (16) des proximalen Gelenkteils (5), im Querschnitt gesehen, mit im wesentlichen V-förmig angeordneten Führungsflächen (25; 30) ausgeführt ist, die zum Zusammenwirken mit im wesentlichen punktförmigen Aufsetzpartien der Stützflächen der Tragköpfe (17, 18; 17', 18') bestimmt sind.

7. Handgelenk nach einem der vorangehenden Ansprüche, dadurch gekennzeichnet, dass der proximale Gelenkteil (5) mit mindestens zwei nebeneinander angeordneten, im Radius (1) verankerbaren stiftförmigen Befestigungselementen (10, 11) versehen ist.

## Claims

1. Artificial hand joint for executing a flexion and an extension movement with a distal joint part (6) securable to the capitate (3), which contains a supporting head (17, 17') with a crowned supporting surface and a proximal joint part (5) securable to the radius (1), which contains a guideway (16) for the purpose of receiving the supporting head (17, 17') with a groove shaped guidance section (16a) extending perpendicular to the longitudinal axis (15) of the radius (1) in the direction of the extension movement, in which the supporting head (17, 17') is guided displaceably in a plane determined by the radius (1) and the ulna (2) characterised in that the distal joint part (6) contains a second supporting head (18, 18') with a second crowned supporting surface arranged alongside the first supporting head (17, 17') in the direction of the extension movement; and in that the guideway (16) of the proximal joint part (5) is executed with a second guidance section (16b) for the purpose of receiving the second supporting head (18, 18').

2. Hand joint in accordance with claim 1 characterised in that the distal joint part (6) has a neck section (20, 20', 21) anchorable by a fastening means (22, 22', 23) to the capitate (3); and in that the two supporting heads (17, 18; 17', 18') are each formed by an at least partly spherical protrusion projecting proximal from the neck section (20, 20', 21).

3. Hand joint in accordance with claim 2 characterised in that the two supporting heads (17, 18) are each formed at a respective extension formed on the neck section (20, 21).

4. Hand joint in accordance with.claim 1 or 2 characterised in that at least one of the two supporting heads (17', 18') is formed by a supporting element itself securable to the capitate (3) - and/or to the neck section (20').

5. Hand joint in accordance with one of the claims 1 to 4 characterised in that the guideway (16) of the proximal joint part (5) is executed, when viewed in cross section, with curved guidance surfaces corresponding to the supporting surfaces of the supporting heads (17, 18).

6. Hand joint in accordance with one of the claims 1 to 4 characterised in that the guideway (16) of the proximal joint part (5) is executed, when viewed in cross section, with guidance surfaces (25; 30) arranged essentially in V formation which are intended to cooperate with essentially punctiform contact parts of the supporting surfaces of the supporting heads (17, 18; 17', 18').

7. Hand joint in accordance with one of the above claims characterised in that the proximal joint part (5) is provided with at least two pin-like securing elements (10, 11) which are arranged side by side and are anchorable in the radius (1).

## Revendications

1. Articulation artificielle de la main pour exécuter un mouvement de flexion et un mouvement d'extension, avec une pièce d'articulation distale (6) pouvant être fixée au grand os (3), qui comporte une tête porteuse (17, 17') avec une face d'appui bombée, et avec une pièce d'articulation proximale (5) pouvant être fixée au radius (1) qui présente une voie de guidage (16) destinée à recevoir la tête porteuse (17, 17') avec un tronçon de guidage (16a) en forme de rainure s'étendant transversalement à l'axe longitudinal (15) du radius (1), dans le sens du mouvement d'extension, dans lequel est guidée d'une manière déplaçable la tête porteuse (17, 17') dans un plan défini par le radius (1) et le cubitus (2),
caractérisée en ce que la pièce d'articulation distale (6) comporte une deuxième tête porteuse (18, 18') disposée dans la direction du mouvement d'extension à côté de la première tête porteuse (17, 17'), avec une deuxième face d'appui bombée, et en ce que la voie de guidage (16) de la pièce d'articulation proximale (5) est réalisée avec un deuxième tronçon de guidage (16b) destiné à recevoir la deuxième tête porteuse (18, 18').

2. Articulation de la main selon la revendication 1, caractérisée en ce que la pièce d'articulation distale (6) présente une partie de col (20, 20', 21) apte à être ancrée au grand os (3) par un moyen de fixation (22, 22', 23), et en ce que les deux têtes porteuses (17, 18 ; 17', 18') sont formées chacune par une saillie dépassant de la partie de col (20, 20', 21) au côté proximal, au moins partiellement en forme de boule.

3. Articulation de la main selon la revendication 2, caractérisée en ce que les deux têtes porteuses (17, 18) sont réalisées chacune à un prolongement rapporté par formage à la partie de col (20, 21).

4. Articulation de la main selon la revendication 1 ou 2, caractérisée en ce qu'au moins l'une des deux têtes porteuses (17', 18') est formée par un élément d'appui pouvant être fixé en tant que tel au grand os (3) - et/ou à la partie de col (20').

5. Articulation de la main selon l'une des revendications 1 à 4, caractérisée en ce que la voie de guidage (16) de la pièce d'articulation proximale (5), vue en section transversale, est réalisée avec des faces de guidage courbées conformément aux faces d'appui des têtes porteuses (17, 18).

6. Articulation de la main selon l'une des revendications 1 à 4, caractérisée en ce que la voie de guidage (16) de la pièce d'articulation proximale (5), vue en section transversale, est réalisée avec des faces de guidage (25 ; 30) disposées sensiblement en forme de V qui sont destinées à coopérer avec des parties d'application sensiblement en forme de points des faces d'appui des têtes porteuses (17, 18 ; 17', 18').

7. Articulation de la main selon l'une des revendications précédentes, caractérisée en ce que la pièce d'articulation proximale (5) est pourvue d'au moins deux éléments de fixation (10, 11) en forme de broche, disposées l'un à côté de l'autre, aptes à être ancrés dans le radius (1).
